(19) Europäisches Patentamt
European Patent Office
Office européen des brevets

(11) Numéro de publication : **0 041 465**
**B1**

(12) # FASCICULE DE BREVET EUROPÉEN

(45) Date de publication du fascicule du brevet : **25.07.84**

(51) Int. Cl.³ : **C 07 D233/80**, A 01 N 43/50

(21) Numéro de dépôt : **81420079.6**

(22) Date de dépôt : **22.05.81**

(54) **Procédé de préparation de carbamoyl-1(dichloro-3,5-phényl)-3-hydantoines et chlorocarbonyl-1(dichloro-3,5-phényl)-3-hydantoïne intermédiaire utilisable pour ce procédé.**

(30) Priorité : **29.05.80 FR 8012238**
**17.04.81 FR 8107962**

(43) Date de publication de la demande :
**09.12.81 Bulletin 81/49**

(45) Mention de la délivrance du brevet :
**25.07.84 Bulletin 84/30**

(84) Etats contractants désignés :
**AT BE CH DE FR GB IT LI LU NL SE**

(56) Documents cités :
**FR-A- 2 120 222**
**FR-A- 2 323 688**
**FR-A- 2 331 559**
**Chemical Abstracts, Vol. 90, 1979 no. 23040k**
**Le dossier contient des informations techniques présentées postérieurement au dépôt de la demande et ne figurant pas dans le présent fascicule.**

(73) Titulaire : **RHONE-POULENC AGROCHIMIE**
**14-20, rue Pierre Baizet**
**F-69009 Lyon (FR)**

(72) Inventeur : **Gros, Georges**
**124, avenue Général Leclerc**
**F-92340 Bourg La Reine (FR)**
Inventeur : **Molin, Marc**
**6, rue de Suffrene**
**F-93330 Neuilly sur Marne (FR)**

(74) Mandataire : **Chrétien, François et al**
**RHONE-POULENC AGROCHIMIE BP 9163**
**F-69263 Lyon Cedex 1 (FR)**

**EP 0 041 465 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet un nouveau procédé de fabrication de carbamoyl-1(dichloro-3,5-phényl)3-hydantoïnes N-substituées ainsi qu'un nouveau produit, le chlorocarbonyle 1(dichloro-3,5-phényl)-3-hydantoïne, utilisable comme nouvel intermédiaire dans la fabrication de ces produits.

Certaines carbamoyl-1(dichloro-3,5-phényl)3-hydantoïnes N-substituées sont connues comme produits phytosanitaires notamment comme fongicides pour l'agriculture. C'est en particulier le cas de l'isopropylcarbamoyl-1(dichloro-3,5-phényl)-3-hydantoïne, communément appelée iprodione, qui fait l'objet des brevets français 2 120 222 et 2 148 868. Ce composé peut être préparé selon divers procédés. Les brevets ci-dessus décrivent une méthode dans laquelle on fait réagir la (dichloro-3,5-phényl)-3-hydantoïne sur le chlorure d'isopropylcarbamyle, ce dernier étant obtenu par condensation de phosgène sur l'isopropylamine. Cependant ce procédé donne des rendements peut satisfaisants pour une exploitation à une échelle industrielle. La demande française FR 2 331 559 décrit la préparation notamment de (N-isopropyl substitué carbamoyle)-1 (dichloro-3,5-phényl)-3 hydantoïne (cf exemple 1 et les suivants) par réaction de la dichloro-3,5-phényl hydantoïne avec un isopropyl isocyanate substitué sur l'isopropyle. Le même document décrit l'obtention de quelques composés analogues par réaction du chlorure de carbamoyle non hétérocyclique correspondant avec la phényl hydantoïne appropriée. Cependant, comme dit précédemment ce procédé, appliqué à la fabrication de l'iprodione, ne donne pas de rendements satisfaisants. Or le développement des fongicides mentionnés ci-dessus rend nécessaire la mise au point d'un procédé permettant l'accès à ces produits dans des conditions encore plus économiques.

La présente invention a pour objet un procédé permettant de répondre à ce besoin. Elle concerne plus spécialement un procédé de préparation, à partir de la (dichloro-3,5-phényl)-3-hydantoïne, d'un composé de formule (I) :

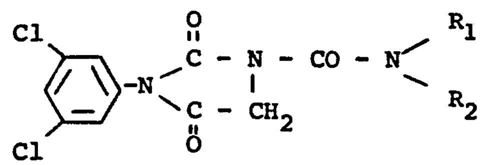

(I)

dans laquelle $R_1$ est un radical alcoyle contenant de 1 à 4 atomes de carbone ou le radical phényle et $R_2$ est un atome d'hydrogène ou un radical alcoyle contenant de 1 à 4 atomes de carbone, caractérisé en ce que :

— dans une première étape, on fait réagir du phosgène sur la (dichloro-3,5-phényl)-3-hydantoïne, en milieu solvant organique inerte, de préférence en présence d'un accepteur d'acide, pour former la chloro carbonyl-1(dichloro-3,5phényl)-3-hydantoïne de formule (II), selon le schéma :

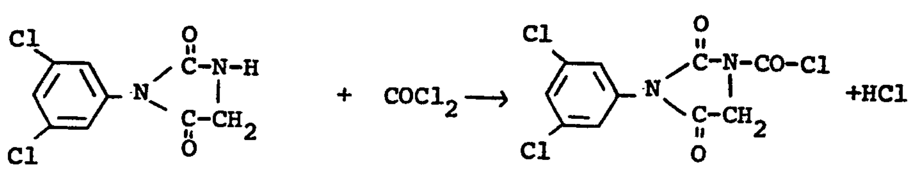

(II)

— et que, dans une seconde étape, on fait réagir le produit de formule (II) obtenu avec une amine primaire ou secondaire de formule $HNR_1R_2$, dans laquelle $R_1$ et $R_2$ ont les significations indiquées ci-dessus, dans un solvant organique inerte, en présence d'un accepteur d'acide, selon le schéma :

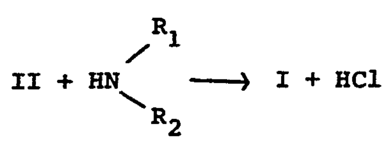

La première étape, dite de phosgénation, s'effectue avec du phosgène, de préférence en excès par rapport à la stœchiométrie, pour éviter la formation de l'urée symétrique résultant de la condensation du composé de formule II sur le composé de départ. Cependant, l'excès ne doit pas être trop important, la partie de phosgène n'ayant pas réagi devant alors être dégazée en fin de réaction. De préférence, on travaille avec un excès de 5 à 100 % en mole.

Cette première étape s'effectue de préférence en présence d'une quantité au moins stœchiométrique, d'un accepteur d'acide, ici d'acide chlorhydrique. Cet accepteur peut être une base organique forte ou minérale.

Comme base organique forte, on peut utiliser une amine tertiaire, telle qu'une trialcoylamine, par exemple la triéthylamine, ou une pyridine telle que la pyridine et la diméthylaminopyridine. Cette base organique est utilisée avantageusement en quantité sensiblement stœchiométrique.

Comme base minérale, on peut utiliser un carbonate alcalin ou un hydroxyde alcalin, la préférence étant donnée au carbonate et en particulier au carbonate disodique. Cette base minérale peut être utilisée en quantité stœchiométrique mais un excès pouvant aller jusqu'à environ 150 % est préféré.

L'accepteur d'acide selon l'invention peut avantageusement être associé à un agent auxiliaire. Ce dernier peut être soit une base auxiliaire, soit un agent de transfert.

La base auxiliaire, associée à raison de 1 à 15 % et de préférence de 5 à 10 %, est une base organique du même type que celle définie précédemment mais différente de la première. On peut notamment utiliser à cet effet la pyridine, la diméthylaminopyridine ou la triéthylamine.

L'agent de transfert utilisable en association avec l'accepteur d'acide, de préférence lorsque celui-ci est une base minérale et notamment un carbonate alcalin, est soit un dérivé d'ammonium ou de phosphonium, soit un agent séquestrant, chacun de ces types de composé étant défini ci-après.

Les dérivés d'ammonium quaternaire utilisables selon la présente invention répondent de préférence à la formule générale :

$$\left[ R_2 - \overset{\displaystyle R_1}{\underset{\displaystyle R_3}{N}} - R_4 \right]_n X$$

dans laquelle :

$R_1$, $R_2$, $R_3$, identiques ou différents, représentent chacun un radical alcoyle contenant de 1 à 20 atomes de carbone, cycloalcoyle contenant de 3 à 6 atomes de carbone, alcényle contenant de 2 à 20 atomes de carbone, phénylalcoyle, éventuellement substitué, dans lequel la partie alcoyle contient de 1 à 6 atomes de carbone,

X représente un atome de chlore, brome ou fluor, ou un radical $SO_4$, $SO_4H$, $PO_4H_2$, hydroxyle, alcoxysulfonyloxyle, contenant de 1 à 4 atomes de carbone (tel que méthoxysulfonyloxyle, éthoxysulfonyloxyle), alcanesulfonyloxyle contenant de 1 à 4 atomes de carbone (tel que méthanesulfonyloxyle ou éthanesulfonyloxyle), arènesulfonyloxyle (tel que benzènesulfonyloxyle ou p.toluènesulfonyloxyle) ou alcanoyloxyle contenant de 1 à 4 atomes de carbone (tel que acétyloxyle et propionyloxyle),

n est un nombre égal à la valence de X.

De bons résultats ont été obtenus en utilisant des mélanges de dérivés d'ammonium quaternaire tels que ceux actuellement commercialisés sous les marques suivantes :

— Adogen 464 : mélange de chlorures de méthyltrialcoylammonium dans lesquels les parties alcoyle contiennent de 8 à 10 atomes de carbone,

— Cemulcat K 012 : mélange de chlorures de dihydroxyéthyle dialcoyle ammonium dans lesquels les parties alcoyle contiennent de 16 à 18 atomes de carbone.

De préférence, on utilise comme catalyseur des dérivés d'ammonium de formule :

$$[R_5—N(C_4H_9)_3]_n X$$

dans laquelle :

X et n ont même signification que dans la formule précédente et,

$R_5$ représente un radical alcoyle contenant de 1 à 4 atomes de carbone, tels que le chlorure d'éthyltributylammonium, le chlorure de méthyltributylammonium, le chlorure de tétrabutylammonium ou le bisulfate de tétrabutylammonium.

Les dérivés de phosphonium utilisables selon la présente invention répondent de préférence à la formule :

$$\left[ R'_2 - \overset{\displaystyle R'_1}{\underset{\displaystyle R'_3}{P}} - R'_4 \right] Y$$

dans laquelle :

$R_1'$, $R_2'$, $R_3'$ et $R_4'$, identiques ou différents, représentent chacun un radical alcoyle contenant de 2 à 8 atomes de carbone et,

Y représente un atome de chlore ou de brome.

La quantité de ces dérivés peut varier dans des limites très larges allant de 0,01 mole à 1 mole par équivalent d'agent basique utilisé. En pratique, pour des raisons économiques, on utilise de 0,5 à 5 % en mole de dérivé de phosphonium par mole de réactif de base.

Les carbonates alcalins peuvent également être avantageusement utilisés en association avec un agent séquestrant en soi connu de formule :

$$N{-}[CHR_1{-}CHR_2{-}O{-}(CHR_3{-}CHR_4{-}O)_n{-}R_5]_3$$

dans laquelle :

n est un nombre entier supérieur ou égal à 0 et inférieur ou égal à environ 10 ($0 \leqslant n \leqslant 10$),

$R_1$, $R_2$, $R_3$ et $R_4$, identiques ou différents, représentent un atome d'hydrogène ou un radical alcoyle ayant de 1 à 4 atomes de carbone et,

$R_5$ représente un radical alcoyle ou cycloalcoyle ayant de 1 à 12 atomes de carbone, un radical phényle ou un radical $-C_mH_{2m}-\varphi$ ou $C_mH_{2m+1}-\varphi-$, où m est compris entre 1 et 12.

De préférence, on utilise un agent séquestrant dans la formule duquel $R_1$, $R_2$, $R_3$ et $R_4$ représentent un atome d'hydrogène ou un radical méthyle, $R_5$ ayant la signification précédente, et de préférence étant un radical alcoyle de 1 à 4 atomes de carbone et n ayant la signification précédente et de préférence étant au moins zéro et au plus 3.

On peut citer :

— la tris(oxa-3 heptyl)amine de formule :

$$N{-}(CH_2{-}CH_2{-}O{-}C_4H_9)_3$$

— la tris(dioxa-3,6 heptyl)amine de formule :

$$N{-}(CH_2{-}CH_2{-}O{-}CH_2{-}CH_2{-}O{-}CH_3)_3$$

— la tris(trioxa-3,6,9 décyl)amine de formule :

$$N{-}(CH_2{-}CH_2{-}O{-}CH_2{-}CH_2{-}O{-}CH_2{-}CH_2{-}O{-}CH_3)_3$$

— la tris(dioxa-3,6 octyl)amine de formule :

$$N{-}(CH_2{-}CH_2{-}O{-}CH_2{-}CH_2{-}O{-}C_2H_5)_3$$

— la tris(trioxa-3,6,9 undécyl)amine de formule :

$$N{-}(CH_2{-}CH_2{-}O{-}CH_2{-}CH_2{-}O{-}CH_2{-}CH_2{-}O{-}C_2H_5)_3$$

— la tris(dioxa-3,6 nonyl)amine de formule :

$$N{-}(CH_2{-}CH_2{-}O{-}CH_2{-}CH_2{-}O{-}C_3H_7)_3$$

— la tris(trioxa-3,6,9 dodécyl)amine de formule :

$$N{-}(CH_2{-}CH_2{-}O{-}CH_2{-}CH_2{-}O{-}CH_2{-}CH_2{-}O{-}C_3H_7)_3$$

— la tris(dioxa-3,6 décyl)amine de formule :

$$N{-}(CH_2{-}CH_2{-}O{-}CH_2{-}CH_2{-}O{-}C_4H_9)_3$$

— la tris(trioxa-3,6,9 tridécyl)amine de formule :

$$N{-}(CH_2{-}CH_2{-}O{-}CH_2{-}CH_2{-}O{-}CH_2{-}CH_2{-}O{-}C_4H_9)_3$$

On peut citer encore :

— la tris(dioxa-3,6 méthyl-4 heptyl)amine de formule :

$$N{-}(CH_2{-}CH_2{-}O{-}\underset{\displaystyle CH_3}{\underset{|}{C}}H{-}CH_2{-}O{-}CH_3)_3$$

— la tris(dioxa-3,6 diméthyl-2,4 heptyl)amine de formule :

$$N-(CH_2-\underset{CH_3}{CH}-O-\underset{CH_3}{CH}-CH_2-O-CH_3)_3$$

La quantité de ces dérivés est telle que leur proportion molaire par rapport à l'accepteur d'acide est comprise entre environ 0,5 et 15 % et de préférence entre 1 et 10 %.

La phosgénation est effectuée dans un solvant organique inerte dans les conditions de réaction. On donnera la préférence aux solvants de point d'ébullition relativement élevé pour permettre un dégazage facile du phosgène en excès, notamment des solvants aromatiques tels que toluène ou xylène, aromatique chlorés comme le chlorobenzène, aliphatiques comme le dodécane ou aliphatiques chlorés tels que le dichloréthane ou le trichloréthane.

On peut effectuer la phosgénation à une température comprise entre 0 °C et la température d'ébullition du milieu réactionnel. Cependant, la réaction étant par nature plutôt lente, il est avantageux de l'accélérer en chauffant, à une température comprise entre 60 et 130 °C, et de préférence à reflux. Lorsqu'on travaille à température élevée, il est avantageux d'opérer en autoclave à des pressions pouvant aller jusqu'à 5 bars (pression absolue).

En pratique, on charge dans le réacteur le solvant et le phosgène, de préférence liquide, et l'hydantoïne de départ. Celle-ci étant peu soluble dans le milieu, on obtient une suspension que l'on chauffe et à laquelle, éventuellement, on ajoute l'accepteur d'acide, pour accélérer la réaction. Le chauffage doit être maintenu suffisamment longtemps pour que la transformation soit complète. On a constaté que des temps de chauffage de 1 à 5 heures conviennent bien.

A la fin de la réaction, deux voies sont possibles. La première consiste à isoler le chlorocarbonyle intermédiaire, dont les caractéristiques sont données dans un exemple ci-après. On constate que le rendement est pratiquement quantitatif.

Cependant, et avantageusement, quand on utilise le procédé industriellement, la solution organique de cet intermédiaire peut être ensuite utilisée telle quelle dans la seconde phase dite de condensation. De toute manière, même dans ce cas, le milieu doit être refroidi et le phosgène en excès dégazé avant de démarrer la seconde étape.

Celle-ci est beaucoup plus rapide que la précédente et s'effectue à une température allant de 0 à 130 °C, de préférence de 20 à 80 °C.

Sur le chlorocarbonyle en milieu organique, on coule des quantités stœchiométriques respectivement de l'amine puis d'un accepteur d'acide, qui peut être de même nature (mais pas nécessairement identique) que celui utilisé dans la première étape ou être l'amine elle-même (auquel cas, elle est engagée deux fois dans la réaction) ou encore une base minérale. Après refroidissement et lavage en milieu acide, puis élimination du solvant, on obtient, avec un excellent rendement qui peut être supérieur à 90 %, le dérivé N-substitué attendu.

Les exemples suivants sont donnés à titre indicatif pour illustrer l'invention.

## Exemple 1

### Première étape

On coule successivement, dans un réacteur pentacol de 1 l muni d'un réfrigérant, 300 ml de chlorobenzène puis, sous agitation, environ 40 g (soit 0,4 mole) de phosgène liquide, et, après arrêt de l'agitation, 61,3 g (soit 0,25 mole) de (dichloro-3,5-phényl)-3-hydantoïne. Le réacteur est alors chauffé à 75 °C pendant 1 heure, le liquide du réfrigérant étant maintenu à une température permettant de condenser le phosgène. Dans le réacteur refroidi à 30 °C environ, on coule, en 10 à 15 mn, 25 g (soit 0,25 mole) de triéthylamine. La température est portée à 130 °C pendant 3 h 30 mn, le liquide du réfrigérant étant maintenu à une température permettant de condenser le solvant et dégazer l'acide chlorhydrique.

### Seconde étape

Après avoir refroidi à 35 °C le milieu obtenu à l'étape précédente, on coule, en 15 mn, 14,7 g (soit 0,25 mole) d'isopropylamine puis 25 g (soit 0,25 mole) de triéthylamine. On chauffe le milieu à 75 °C pendant 20 mn puis on refroidit à 20 °C. On coule ensuite 300 ml d'acide sulfurique à 5 % et on agite énergiquement pendant 30 minutes. Après décantation de la phase aqueuse, on lave la phase chlorobenzénique à l'eau distillée. La couche chlorobenzénique est séchée sur sulfate de soude anhydre puis concentrée sous pression réduite. On obtient 80 g, soit un rendement de 97 %, d'un produit de point de fusion 130 °C contenant 91,5 % d'isopropylcarbamoyl 1(dichloro-3,5 phényl)-3)hydantoïne.

## Exemple 2

On opère comme à la première phase de l'exemple 1, si ce n'est qu'en fin de réaction, le produit intermédiaire est isolé. On obtient un solide blanc de point de fusion égal à 202 °C et ayant la composition centésimale suivante :

| % | C | H | Cl | N | O |
|---|---|---|---|---|---|
| calculé | 39,06 | 1,64 | 34,58 | 9,11 | 15,61 |
| trouvé | 39,09 | 1,45 | 34,34 | 9,18 | 15,62 |

L'analyse en spectrographie infra-rouge montre l'existence des bandes à 1 830-1 820, 1 760 et 1 720 $cm^{-1}$, caractéristiques des groupes carbonyle, correspondant au chlorocarbonyle 1(dichloro-3,5-phényl)-3-hydantoïne.

Exemples 3 à 7

On opère comme à l'exemple 1, en remplaçant, dans la seconde étape, l'isopropylamine par une quantité équivalente respectivement d'éthylamine, de n-propylamine, d'aniline et de diméthylamine. Les rendements et points de fusion des produits obtenus sont indiqués dans le tableau suivant :

| $HNR_1R_2$ Amine employée | Point de fusion | Rendement |
|---|---|---|
| n-propylamine | 92°C | 99 % |
| phénylamine | 182°C | 99 % |
| éthylamine | 152°C | 97 % |
| N,N-diméthylamine | 162°C | 98 % |

Exemple 8

Utilisation de l'isopropylamine comme accepteur d'acide.

A une suspension de 9,2 g (0,03 mole) de chlorocarbonyle 1(dichloro-3,5-phényl)-3-hydantoïne obtenue à l'exemple 2, dans 80 ml de chlorobenzène, on coule, en 20 mn, une solution de 3,6 g d'isopropylamine (soit 0,06 mole) dans 20 ml de chlorobenzène en maintenant la température à au plus 30 °C. Après une heure d'agitation, on ajoute 150 ml de chlorobenzène. La solution organique est lavée avec 100 ml d'une solution aqueuse d'acide chlorhydrique à 3,6 %. Après séchage et concentration sous pression réduite, on obtient 9,9 g d'un solide fondant à 135 °C et correspondant à l'isopropylcarbamoyl 1(dichloro-3,5-phényl)-3-hydantoïne.

Exemples 9 à 13

On coule, à une température de 20 à 30 °C, une mole d'une base organique (avec éventuellement un excès) sur une suspension d'une mole de (dichloro-3,5-phényl)-3-hydantoïne dans un mélange préalablement préparé de 1,5 mole de phosgène dans 1 100 ml de chlorobenzène. On chauffe ensuite le milieu réactionnel pendant un certain temps. Dans l'un des essais, la phosgénation a été effectuée dans un autoclave à une pression d'environ 3 bars. On isole le chlorocarbonyle obtenu. On l'utilise ensuite dans la réaction de condensation telle que décrite à l'exemple 1.

Les conditions variables de la phosgénation ainsi que les rendements en chlorocarbonyle intermédiaire et en isopropyl-1 (dichloro-3,5-phényl)-3 hydantoïne sont consignés dans le tableau suivant :

(Voir Tableau page suivante)

| Exemple No | accepteur d'acide | base auxiliaire | température | durée de chauffage en h | rendement en % molaire/réactif | |
|---|---|---|---|---|---|---|
| | | | | | en chlorocarbonyle | en iprodione |
| 9 | TEA | - | 80°C | 12 | 91 | 86 |
| 10 | DMAP | - | 100°C | 5 | 91 | 82 |
| 11 | TEA 90 % | DMAP (10%) | 80°C | 5 | 81 | - |
| 12 | Pyridine (10% d'excès) | - | 70-75°C | 10 | 88 | - |
| 13 | Pyridine (sous pression) | - | 120-130°C | 1 | 93 | 74 |

TEA = triéthylamine
DMAP = diméthylaminopyridine.

## Exemple 14

a) Phosgénation

On introduit, à 20 °C, dans un réacteur équipé d'un réfrigérant :

— 1 mole de (dichloro-3,5-phényl)-3-hydantoïne,
— 2 moles de carbonate disodique,
— 0,09 mole de diméthylaminopyridine et,
— 1 100 ml de chlorobenzène.

Puis on coule 2 moles de phosgène.

On chauffe ensuite le mélange réactionnel à 80 °C pendant 1 heure 30 minutes puis à 105 °C pendant une durée égale. On filtre à chaud (110 °C) les minéraux et on lave le gâteau par 2 fois 50 ml de chlorobenzène chaud (130 °C). On obtient une suspension de chlorocarbonyl-1(dichloro-3,5-phényl)-3-hydantoïne, avec un rendement en ce composé de 94 %.

b) Condensation

Celle-ci est effectuée en suivant le mode opératoire de l'exemple 1 (seconde étape).

Dans ces conditions, on obtient de l'isopropyl carbamoyl-1 (dichloro-3,5 phényl)-3-hydantoïne avec un rendement de 92 %.

## Exemple 15

a) Phosgénation

Dans un réacteur équipé d'un réfrigérant, on introduit :

— 1 mole de (dichloro-3,5-phényl)-3-hydantoïne,
— 2,5 moles de carbonate disodique,
— 0,036 mole de tétrabutylammonium bisulfate,
— 1 100 ml de chlorobenzène.

On coule ensuite 4 moles de phosgène et on chauffe le mélange réactionnel à 80 °C pendant 1 heure 30 minutes, puis à 105 °C pendant 1 heure 30 minutes. On filtre à chaud (environ 110 °C) la totalité des minéraux en suspension et on lave le gâteau par 2 fois 50 ml de chlorobenzène à 130 °C.

b) Condensation

Celle-ci est effectuée en suivant le mode opératoire de l'exemple 1 (seconde étape). Le produit

résultant est isolé par évaporation du solvant après lavage acide et neutre de la solution chlorobenzénique.

Dans ces conditions, on obtient, avec un rendement de 92 %, un produit contenant 94 % d'isopropylcarbamoyl-1 (dichloro-3,5-phényl)-3-hydantoïne.

Si on répète cet essai en utilisant dans la phosgénation 0,018 mole au lieu de 0,036 de bisulfate, on obtient l'isopropylcarbamoyl-1 (dichloro-3,5 phényl)-3-hydantoïne avec un rendement de 80 %.

**Revendications**

1. Procédé de préparation, à partir de la (dichloro-3,5-phényl)-3-hydantoïne, d'un composé de formule (I) :

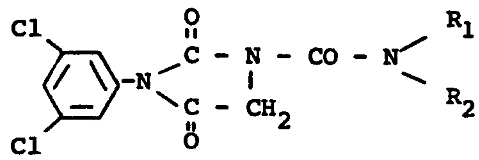

(I)

dans laquelle $R_1$ est un radical alcoyle contenant de 1 à 4 atomes de carbone ou le radical phényle et $R_2$ est un atome d'hydrogène ou un radical alcoyle contenant de 1 à 4 atomes de carbone, caractérisé en ce que :

— dans une première étape, on fait réagir du phosgène sur la (dichloro-3,5-phényl)-3-hydantoïne, en milieu solvant organique inerte, en présence d'un accepteur d'acide, pour former la chlorocarbonyle 1(dichloro-3,5-phényl)-3-hydantoïne de formule (II), selon le schéma :

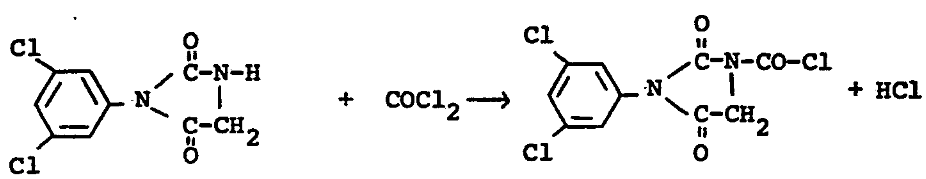

(II)

— et que, dans une seconde étape, on fait réagir le produit de formule (II) obtenu avec une amine primaire ou secondaire de formule $HNR_1R_2$, dans laquelle $R_1$ et $R_2$ ont les significations indiquées ci-dessus, dans un solvant organique inerte, en présence d'un accepteur d'acide, selon le schéma :

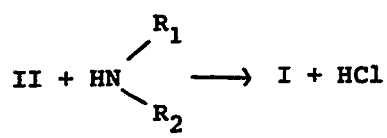

2. Procédé selon la revendication 1, caractérisé en ce que les deux étapes de la réaction sont effectuées à une température comprise entre 0 °C et la température d'ébullition du mélange réactionnel.

3. Procédé selon l'une des revendications 1 et 2, caractérisé en ce que l'accepteur d'acide est une amine tertiaire.

4. Procédé selon la revendication 3, caractérisé en ce que l'accepteur d'acide est la triéthylamine.

5. Procédé selon la revendication 3, caractérisé en ce que l'accepteur d'acide est la pyridine.

6. Procédé selon l'une des revendications 1 et 2, caractérisé en ce que l'accepteur d'acide est une base minérale.

7. Procédé selon la revendication 6, caractérisé en ce que la base minérale est un carbonate alcalin.

8. Procédé selon l'une des revendications 1 à 7, caractérisé en ce que l'accepteur d'acide est associé à 0,5 à 15 % en mole, par rapport à l'accepteur d'acide, d'un agent auxiliaire choisi dans le groupe comprenant une base auxiliaire organique forte différente de l'accepteur d'acide, un agent de transfert du type dérivé d'ammonium quaternaire, dérivé de phosphonium ou agent sequestrant.

9. Procédé selon la revendication 8, caractérisé en ce que la base auxiliaire est choisie dans le groupe comprenant la triéthylamine, la pyridine et la diméthylaminopyridine.

10. Procédé selon la revendication 8, caractérisé en ce que l'agent de transfert est un dérivé d'ammonium quaternaire de formule générale :

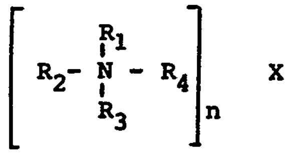

dans laquelle :

$R_1$, $R_2$, $R_3$, $R_4$, identiques ou différents, représentent chacun un radical alcoyle contenant de 1 à 20 atomes de carbone, cycloalcoyle contenant de 3 à 6 atomes de carbone, alcényle contenant de 2 à 20 atomes de carbone, hydroxyalcoyle contenant de 1 à 20 atomes de carbone, phénylalcoyle, éventuellement substitué, dans lequel la partie alcoyle contient de 1 à 6 atomes de carbone,

X représente un atome de chlore, brome ou fluor, ou un radical $SO_4$, $SO_4H$, $PO_4H_2$, hydroxyle, alcoxylsulfonyloxyle, contenant de 1 à 4 atomes de carbone, alcanesulfonyloxyle contenant de 1 à 4 atomes de carbone, arènesulfonyloxyle ou alcanoyloxyle contenant de 1 à 4 atomes de carbone,

n est un nombre égal à la valence de X.

11. Procédé selon la revendication 10, caractérisé en ce que le dérivé d'ammonium quaternaire est le chlorure ou le bisulfate de tétrabutylammonium.

12. Procédé selon la revendication 8, caractérisé en ce que l'agent de transfert est un dérivé de phosphonium de formule :

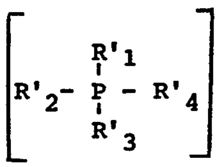 Y

dans laquelle :

$R_1'$, $R_2'$, $R_3'$ et $P_4'$, identiques ou différents, représentent chacun un radical alcoyle contenant de 2 à 8 atomes de carbone et,

Y représente un atome de chlore ou de brome.

13. Procédé selon la revendication 8, caractérisé en ce que, lorsque l'accepteur d'acide est une base minérale ou un carbonate alcalin, l'agent de transfert est un agent sequestrant de formule :

$$N[-CHR_1-CHR_2-O-(CHR_3-CHR_4-O)_n-R_5]_3$$

dans laquelle :

n est un nombre entier supérieur ou égal à 0 et inférieur ou égal à environ 10 ($0 \leq n \leq 10$),

$R_1$, $R_2$, $R_3$ et $R_4$, identiques ou différents, représentent un atome d'hydrogène ou un radical alcoyle ayant de 1 à 4 atomes de carbone, et

$R_5$ représente un radical alcoyle ou cycloalcoyle ayant de 1 à 12 atomes de carbone, un radical phényle ou un radical $-C_mH_{2m}-\varphi$ ou $C_mH_{2m+1}-\varphi-$, où m est compris entre 1 et 12.

14. Procédé selon la revendication 13, caractérisé en ce que la base minérale est associée à la (tris-(dioxa-3,6-heptyl)amine.

15. Procédé selon l'une des revendications 1 et 2, caractérisé en ce que, dans la seconde étape, on utilise également l'amine $HNR_1R_2$ comme accepteur d'acide.

16. Procédé selon l'une des revendications 1 à 15, caractérisé en ce que le solvant est un solvant aromatique.

17. Procédé selon la revendication 16, caractérisé en ce que le solvant est le chlorobenzène.

18. Procédé selon l'une des revendications 1 à 15, caractérisé en ce que le solvant est un solvant aliphatique.

19. Procédé selon la revendication 2, caractérisé en ce que la première étape est effectuée à une température de 60 °C à 130 °C.

20. Procédé selon la revendication 19, caractérisé en ce que la réaction est effectuée sous une pression absolue de 1 à 5 bars.

21. Procédé selon la revendication 2, caractérisé en ce que la seconde étape est effectuée à une température comprise de 0 °C à 130 °C.

22. Procédé selon l'une des revendications 1 à 21, caractérisé en ce que les deux étapes sont effectuées en continu.

23. Procédé selon l'une des revendications 1 à 21, caractérisé en ce que, à la fin de la première étape, on sépare le composé intermédiaire.

24. A titre de produit nouveau, la chlorocarbonyle-1(dichloro-3,5-phényl)-3-hydantoïne.

**Claims**

1. A process for the preparation, starting from 3-(3,5-dichlorophenyl)-hydantoin, of a compound of the formula (I) :

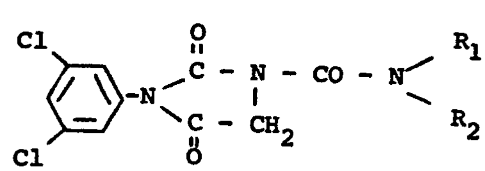 (I)

in which $R_1$ is an alkyl radical containing from 1 to 4 carbon atoms or the phenyl radical and $R_2$ is a hydrogen atom or an alkyl radical containing from 1 to 4 carbon atoms, which comprises :

— in a first step, reacting phosgene with 3-(3,5-dichlorophenyl)-hydantoin in an inert organic solvent medium, in the presence of an acid acceptor, in order to form 1-chlorocarbonyl-3-(3,5-dichlorophenyl)-hydantoin, of the formula (II), in accordance with the equation :

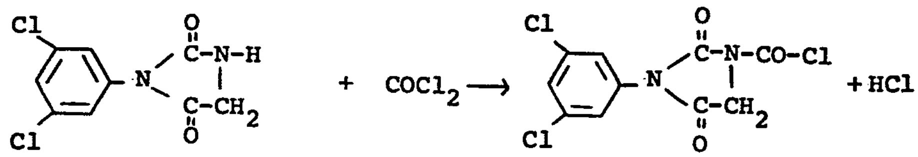

(II)

— and, in a second step, reacting the resulting product, of the formula (II) with a primary or secondary amine of the formula $HNR_1R_2$, in which $R_1$ and $R_2$ have the meanings indicated above, in an inert organic solvent, in the presence of an acid acceptor, in accordance with the equation :

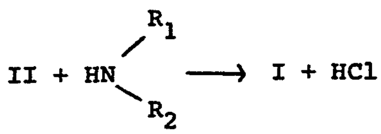

2. A process according to claim 1, wherein the two steps of the reaction are carried out at a temperature between 0 °C and the b. p. of the reaction mixture.

3. A process according to one of claims 1 and 2, wherein the acid acceptor is a tertiary amine.

4. A process according to claim 3, wherein the acid acceptor is triethylamine.

5. A process according to claim 3, wherein the acid acceptor is pyridine.

6. A process according to one of claims 1 and 2, wherein the acid acceptor is an inorganic base.

7. A process according to claim 6, wherein the inorganic base is an alkali metal carbonate.

8. A process according to one of claims 1 to 7, wherein the acid acceptor is associated with 0,5 to 15 mol % of an auxiliary agent chosen in the group comprising an auxiliary strong organic base which is different from the acid acceptor, a transfer agent such as quaternary ammonium derivative, phosphonium derivative or sequestering agent.

9. A process according to claim 8, wherein the auxiliary base is chosen from the group comprising triethylamine, pyridine and dimethylaminopyridine.

10. A process according to claim 8, wherein the transfer agent is a quaternary ammonium derivative of the general formula :

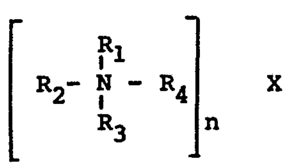

in which :

$R_1$, $R_2$, $R_3$ and $R_4$, which are identical or different, each represent an alkyl radical containing from 1 to 20 carbon atoms, a cycloalkyl radical containing from 3 to 6 carbon atoms, an alkenyl radical containing from 2 to 20 carbon atoms, a hydroxyalkyl radical containing from 1 to 20 carbon atoms or an optionally substituted phenylalkyl radical in which the alkyl part contains from 1 to 6 carbon atoms,

X represents a chlorine, bromine or fluorine atom or an $SO_4$, $SO_4H$, $PO_4H_2$ or hydroxyl radical, an alkoxysulphonyloxy radical containing from 1 to 4 carbon atoms, an alkanesulphonyloxy radical containing from 1 to 4 carbon atoms, an arenesulphonyloxy radical, or an alkanoyloxy radical containing from P to 4 atoms and

n is a number equal to the valency of X.

11. A process according to claim 10, wherein the quaternary ammonium derivative is tetrabutylammonium chloride or bisulphate.

12. A process according to claim 8, wherein the transfer agent is a phosphonium derivative of the formula :

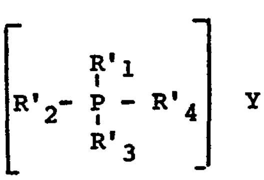

in which :

$R_1'$, $R_2'$, $R_3'$ and $R_4'$, which are identical or different, each represent an alkyl radical containing from 2 to 8 carbon atoms and

Y represents a chlorine or bromine atoms.

13. A process according to claim 8, when the acid acceptor is an inorganic base or an alkaline carbonate, wherein the transfert agent is a sequestering agent of the formula :

$$N—[CHR_1—CHR_2—O—(CHR_3—CHR_4—O)_n—R_5]_3$$

in which :

n is an integer which is greater than or equal to 0 and less than or equal to about 10 ($0 \leq n \geq 10$),

$R_1$, $R_2$, $R_3$ and $R_4$, which are identical or different, represent a hydrogen atom or an alkyl radical having from 1 to 4 carbon atoms, and $R_5$ represents an alkyl or cycloalkyl radical having from 1 to 12 carbon atoms, a phenyl radical or a radical $—C_mH_{2m}—\varphi$ or $C_mH_{2m+1}—\varphi—$, in which m is between 1 and 12.

14. A process according to claim 13, wherein the inorganic base is associated with tris-(3,6-dioxaheptyl)-amine.

15. A process according to one of claims 1 and 2, wherein, in the second step, the amine $HNR_1R_2$ is also used as the acid acceptor.

16. A process according to one of claims 1 to 15, wherein the solvent is an aromatic solvent.

17. A process according to claim 16, wherein the solvent is chlorobenzene.

18. A process according to one of claims 1 to 15, wherein the solvent is an aliphatic solvent.

19. A process according to claim 2, wherein the first step is carried out at a temperature of 60 °C to 130 °C.

20. A process according to claim 19, wherein the reaction is carried out under an absolute pressure of 1 to 5 bars.

21. A process according to claim 2, wherein the second step is carried out at a temperature of 0 °C to 130 °C.

22. A process according to one of claims 1 to 21, wherein the two steps are carried out continuously.

23. A process according to one of claims 1 to 21, wherein the intermediate is separated off at the end of the first step.

24. By way of a new product, 1-chlorocarbonyl-3-(3,5-dichlorophenyl)-hydantoin.

**Ansprüche**

1. Verfahren zur Herstellung einer Verbindung der Formel :

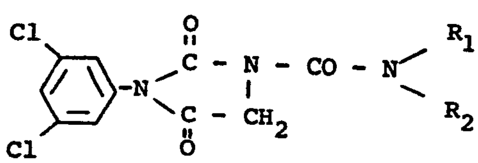

(I)

worin $R_1$ ein Alkylrest mit 1 bis 4 Kohlenstoffatomen oder der Phenylrest ist, und $R_2$ ein Wasserstoffatom oder einen Alkylrest mit 1 bis 4 Kohlenstoffatomen bedeutet, ausgehend von 3-(3,5-Dichlor-phenyl)-hydantoin, dadurch gekennzeichnet,

— daß man in einer ersten Stufe Phosgen mit 3-(3,5-Dichlor-phenyl)-hydantoin in einem inerten organischen Lösungsmittelmilieu in Gegenwart eines Säureakzeptors reagieren läßt, um das 1-Chlorcarbonyl-3-(3,5-dichlor-phenyl)-hydantoin der Formel (II) gemäß dem Schema :

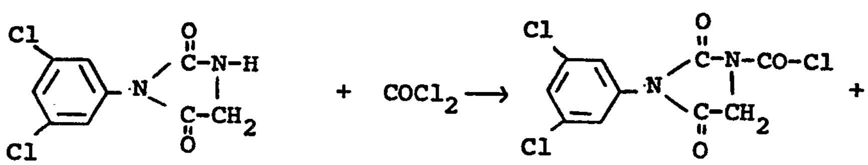

(II)

zu bilden und daß man in einer zweiten Stufe das erhaltene Produkt mit der Formel (II)

— einem primären oder sekundären Amin der Formel $HNR_1R_2$, worin $R_1$ und $R_2$ die angegebenen Bedeutungen besitzen, in einem inerten organischen Lösungsmittel in Gegenwart eines Säureakzeptors umsetzt nach dem Schema :

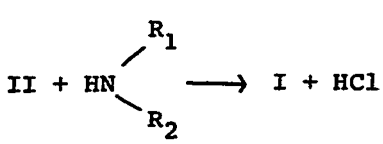

2. Verfahren gemäß Anspruch 1, dadurch gekennzeichnet, daß die zwei Stufen der Reaktion bei einer Temperatur zwischen 0 °C und der Siedetemperatur des Reaktionsgemisches durchgeführt werden.

3. Verfahren nach den Ansprüchen 1 und 2, dadurch gekennzeichnet, daß der Säureakzeptor ein tertiäres Amin ist.

4. Verfahren gemäß Anspruch 3, dadurch gekennzeichnet, daß der Säureakzeptor Triäthylamin ist.

5. Verfahren gemäß Anspruch 3, dadurch gekennzeichnet, daß der Säureakzeptor Pyridin ist.

6. Verfahren gemäß einem der Ansprüche 1 und 2, dadurch gekennzeichnet, daß der Säureakzeptor eine Mineralbase ist.

7. Verfahren gemäß Anspruch 6, dadurch gekennzeichnet, daß die Mineralbase ein Alkalicarbonat ist.

8. Verfahren gemäß einem der Ansprüche 1 bis 7, dadurch gekennzeichnet, daß der Säureakzeptor mit 0,5 bis 15 Mol-% Hilfsmittels assoziiert ist, der aus der Gruppe umfassend eine von dem Säureakzeptor verschiedene starke organische Hilfsbase, ein Übergangsmittel wie ein quaternäres Ammoniumderivat, ein Phosphoniumderivat oder ein Sequestriermittel ausgewählt ist.

9. Verfahren gemäß Anspruch 8, dadurch gekennzeichnet, daß die Hilfsbase ausgewählt ist aus der Gruppe umfassend Triäthylamin, Pyridin und Dimethylaminopyridin.

10. Verfahren gemäß Anspruch 8, dadurch gekennzeichnet, daß das Übergangsmittel ein quaternäres Ammoniumderivat allgemeinen Formel :

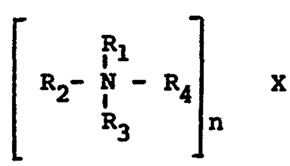

ist, worin bedeuten :

$R_1$, $R_2$, $R_3$, $R_4$, welche identisch oder voneinander verschieden sind, jeweils einen Alkylrest mit 1 bis 20 Kohlenstoffatemen, Cycloalkyl mit 3 bis 6 Kohlenstoffatomen, Alkenyl mit 2 bis 20 Kohlenstoffatomen, Hydroxyalkyl mit 1 bis 20 Kohlenstoffatomen, Phenylalkyl, gegebenenfalls substituiert, wobei der Alkylteil 1 bis 6 Kohlenstoffatome enthält,

X ein Chlor-, Brom- oder Fluoratom oder einen Rest $SO_4$, $HSO_4$, $H_2PO_4$, Hydroxyl, Alkylsulfonyloxyl mit 1 bis 4 Kohlenstoffatomen, Alkansulfonyloxyl mit 1 bis 4 Kohlenstoffatomen, Arylsulfonyloxyl oder Alkanoyloxyl mit 1 bis 4 Kohlenstoffatomen,

n eine ganze Zahl gleich der Valenz von X.

11. Verfahren gemäß Anspruch 10, dadurch gekennzeichnet, daß das quaternäre Ammoniumderivat Tetrabutylammoniumchlorid oder -bisulfat ist.

12. Verfahren gemäß Anspruch 8, dadurch gekennzeichnet, daß das Übergangsmittel ein Phosphoniumderivat der Formel :

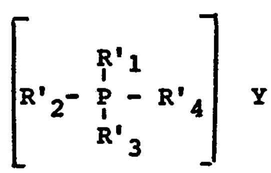

ist, worin

$R_1'$, $R_2'$, $R_3'$, und $R_4'$, welche identisch oder voneinander verschieden sind, jeweils einen Alkylrest mit 2 bis 8 Kohlenstoffatomen bedeuten und

Y ein Chlor- oder Bromatom darstellt.

13. Verfahren gemäß Anspruch 8, dadurch gekennzeichnet, daß, wenn der Säureakzeptor eine Mineralbase oder ein Alkalicarbonat ist, das Übergangsmittel ein Sequestriermittel der Formel :

$$N—[CHR_1—CHR_2—O—(CHR_3—CHR_4—O)_n—R_5]_3$$

ist, worin bedeuten :

n eine ganze Zahl größer oder gleich Null und unterhalb oder gleich etwa 10 ($0 \leq n \leq 10$),

$R_1$, $R_2$, $R_3$ und $R_4$, welche identisch oder voneinander verschieden sind, ein Wasserstoffatom oder einen Alkylrest mit 1 bis 4 Kohlenstoffatomen, und

$R_5$ einen Alkyl- oder Cycloalkylrest mit 1 bis 12 Kohlenstoffatomen, einem Phenylrest oder einen Rest $C_mH_{2m}—\varphi—$ oder $C_mH_{2m+1}—\varphi—$, wobei m zwischen 1 und 12 beträgt.

14. Verfahren gemäß Anspruch 13, dadurch genkennzeichnet, daß die Mineralbase mit Tris (3,6-dioxaheptyl) amin assoziiert ist.

15. Verfahren nach einem der Ansprüche 1 und 2, dadurch gekennzeichnet, daß man in der zweiten Stufe das Amin $HNR_1R_2$ auch als Säureakzeptor verwendet.

16. Verfahren gemäß einem der Ansprüche 1 bis 15, dadurch gekennzeichnet, daß das Lösungsmittel ein aromatisches Lösungsmittel ist.

17. Verfahren gemäß Anspruch 16, dadurch gekennzeichnet, daß das Lösungsmittel Chlorbenzol ist.

18. Verfahren gemäß einem der Ansprüche 1 bis 15, dadurch gekennzeichnet, daß das Lösungsmittel ein aliphatisches Lösungsmittel ist.

19. Verfahren gemäß Anspruch 2, dadurch gekennzeichnet, daß die erste Stufe bei einer Temperatur von 60 bis 130 °C bewirkt wird.

20. Verfahren gemäß Anspruch 19, dadurch gekennzeichnet, daß die Reaktion unter einem absoluten Druck von 1 bis 5 bar bewirkt wird.

21. Verfahren gemäß Anspruch 2, dadurch gekennzeichnet, daß die zweite Stufe bei einer Temperatur zwischen 0 und 130 °C durchgeführt wird.

22. Verfahren nach einem der Ansprüche 1 bis 21, dadurch gekennzeichnet, daß die beiden Stufen kontinuierlich bewirkt werden.

23. Verfahren nach einem der Ansprüche 1 bis 21, dadurch gekennzeichnet, daß man am Ende der ersten Stufe das Zwischenprodukt abtrennt.

24. Als neue Verbindung das 1-Chlorcarbonyl-3-(3,5-dichlorphenyl)-hydantoin.